Europäisches Patentamt

European Patent Office

Office européen des brevets

(19)

(11) Publication number: **0 358 303**
**A1**

## (12) EUROPEAN PATENT APPLICATION

(21) Application number: 89305786.9

(51) Int. Cl.5: **A61N 1/365 , A61B 5/04**

(22) Date of filing: 08.06.89

(30) Priority: 08.06.88 US 204051

(43) Date of publication of application:
14.03.90 Bulletin 90/11

(84) Designated Contracting States:
DE FR GB IT NL SE

(71) Applicant: MEDTRONIC, INC.
7000 Central Avenue N.E.
Minneapolis Minnesota 55432(US)

(72) Inventor: Huberty, Kenneth P.
13175 Kerry St N.W.
Coon Rapids Minnesota 55433(US)
Inventor: Olson, Walter H.
16 Hay Camp Road
North Oaks Minnesota 55127(US)

(74) Representative: Tomlinson, Kerry John et al
Frank B. Dehn & Co. European Patent
Attorneys Imperial House 15-19 Kingsway
London WC2B 6UZ(GB)

(54) Apparatus for detecting and treating tachyarrhythmia for a pacer, cardioverter, defibrillator.

(57) The cardiac cycle length of the heart in a slow supraventricular rhythm (240) is used to establish and automatically adapt the rate detection threshold (210) for a tachyarrhythmia detector A timing window (W1) is established extending from the end of a blanking interval (200) to the end of a fibrillation detection interval (210) and sensed ventricular events falling within the window are counted. The lower (211) and upper (212) limits of the fibrillation detection interval are physician-programmable.

FIG 2

## METHOD AND APPARATUS FOR DETECTING TACHYARRHYTHMIA FOR A PACER, CARDIOVERTER, DEFIBRILLATOR

The invention relates to implantable medical devices and more particularly to pacer, cardioverter, defibrillator devices which automatically detect and treat episodes of tachycardia and/or fibrillation.

Implantable devices which can detect and treat ventricular tachycardias are known in the art from U.S. Patent No. 4,384,585 to D. Zipes and U.S. Patent No. 4,493,325 to P. Beckman et al. Each of these devices utilizes a sense amplifier for monitoring the time period between a series of ventricular events. The measured cycle lengths (CL) are compared with a fixed but programmable threshold interval to ascertain if the cycle lengths of the current rhythm are pathologic.

However, since heart rates associated with normal sinus rhythm can overlap heart rates associated with pathologic tachycardias, rate threshold information is usually only one of a number of criteria used to decide whether a particular collection of cardiac cycles represents a treatable arrhythmia.

For example, the investigational pacer, cardioverter, defibrillator, Model 7215 manufactured by Medtronic, Inc., Minneapolis, U S A, may combine rate information with "onset" and/or "stability" criteria to help separate pathologic arrhythmias from normal rhythms.

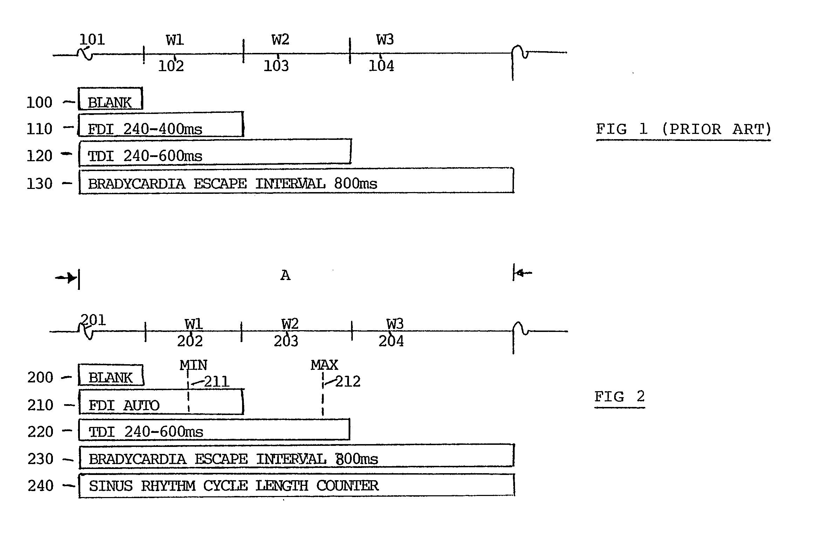

This prior art device sets up detection windows following each ventricular event. As shown in Fig. 1, there is a brief 120 ms blanking interval 100 after a ventricular sense event 101. A software timer is used to generate a fibrillation detection interval 110 (FDI). This timer 110 is started by the ventricular sense event, and the time interval extending from the end of the blanking period to the end of the FDI is a timing window 102 referred to herein as W1.

A ventricular sense event which occurs in W1 is potentially a result of a tachyarrhythmia. The 7215 counts the number of beats falling within these W1 intervals. A sequence of such events may be collected and an episode of ventricular tachycardia is declared and a therapeutic treatment routine is begun.

Returning to Fig. 1, there is another timer which is used to set up a tachycardia detection interval 120 (TDI). This fixed but programmable interval defines a second detection window 103 called W2 herein. Cardiac events which occur in this window may be the result of ventricular tachycardia. If a fixed but programmable number of such beats is collected, the device declares that the patient is in a ventricular tachyarrhythmia. Once again, the device will enter into an appropriate therapeutic treatment routine.

Events which fall within window W3 are considered normal events, and they resynchronize the bradycardia escape interval timer 130 in a known manner.

## SUMMARY OF THE INVENTION

In contrast to the Model 7215 operation described, the present invention provides a variable or automatically self-adapting fibrillation detection interval. This interval is called FDI AUTO in the figures. The value of FDI AUTO is updated periodically and is selected as a function of the historic cardiac cycle length of the preceding supraventricular rhythm. Typically, the preceding rhythm will originate in the sinus node. In operation, the device detects a sequence of ventricular events and computes a representative supraventricular rhythm cycle length called SRCL AVE. This interval is used to compute the FDI AUTO. Both the SRCL AVE and FDI AUTO are periodically updated while the heart is in a slow supraventricular rhythm. The maximum and minimum length of the FDI AUTO interval are physician-programmable.

By setting the FDI AUTO as a function of the SRCL AVE, the device exploits the observation that there is a strong correlation between the cycle length of a tachyarrhythmia and the cycle length of the preceding supraventricular rhythm. The method of selecting the representative SRCL AVE and the computation of the related FDI AUTO is set forth below.

An embodiment of the invention will now be described by way of example and with reference to the accompanying drawings.

## BRIEF DESCRIPTION OF THE DRAWINGS

Fig. 1 is a timing diagram of the detection operation of a prior art pacer, cardioverter, defibrillator.
Fig. 2 is a timing diagram of the detection operation of the present invention.

Fig. 3 is a timing diagram depicting several sequential cardiac events which is useful in illustrating the computation of the variable fibrillation detection interval.

Fig. 4 is a diagram depicting the computation of the SRCL AVE and FDI AUTO.

Modern pacer, cardioverter, defibrillators,such as the Medtronic Model 7215, are microprocessor based, implantable medical devices which utilize both hardware and software to implement the timers and counters required to carry out the detection operation. In these devices, one or more therapeutic regimes are entered if the device detects a treatable arrhythmia based upon the detection operation. The present invention is directed to an improved method for detecting tachyarrhythmia and more particularly to detect fibrillation. The specific structures which can be used to implement this improved method are a matter of routine engineering choice, and suitable structures are apparent to one of ordinary skill in the art and are also known from U.S. Patent No. 4,493,325. At present, the invention is contemplated for use in devices like the Model 7215 which sense and electrically treat the ventricle. However, the tachyarrhythmia detector of the present invention may be useful in atrial applications and may be useful to invoke drug therapies of the heart as well.

Turning to the prior art detection method depicted in Fig. 1, there is shown an intracardiac electrogram which is detected upon the ventricular lead of the device. A ventricular sense event 101 initiates a blanking interval 100. Blanking refers to a time interval during which the device will be unresponsive to signals present on the ventricular lead. There are a variety of ways of achieving blanking within implanted medical devices, and typically the output of the ventricular sense amplifier is disabled for the blanking interval by a dedicated blanking timer. After the blanking interval ends, the ability to sense the intracardiac electrogram is restored in the device.

In the prior art device, the ventricular sense event 101 also starts a fibrillation detection interval timer which times out a physician-programmable time interval. The portion of this time interval which extends beyond the conclusion of the blanking interval constitutes a detection window 102 for fibrillation events. This interval is depicted on the diagram as the window W1. If a sufficient number of events fall within W1 windows, a fibrillation episode may be declared, and the prior art device enters a treatment regime.

In the present invention, this fibrillation detection interval 210 is variable between a physician-programmable maximum value 212 and a programmable minimum value 211 as shown in Fig. 2. However, the instantaneous value of the interval is automatically set as a function of the patient's most recent supraventricular rhythm. It is contemplated that the physician will select both an upper and lower bound for the fibrillation detection intervals and will also select a slope value to tailor the detector to an individual patient. It is also contemplated that the physician may select a nominal value for FDI AUTO which may be extended or reduced by a physician-selected amount. For example, the physician may select a 400 ms nominal interval which may be extended or reduced by 100 ms so that FDI AUTO may vary from 300 ms to 500 ms in duration.

Another alternative contemplated includes the selection of a programmable value for the FDI which may then be extended or reduced by a programmable percentage or a programmable fraction of the value.

The cycle length of the preceding rhythm is monitored in the invention by a timer 240 which keeps track of the time interval between successive sensed events and/or the time interval between pace and sense events. Cardiac cycles which end in a ventricular pace event are preferably excluded from the computation of a representative supraventricular rhythm cycle length. Cycles which result in a pace event indicate that the bradycardia escape interval timer 230 has timed out, and such cycles do not reflect a physiologic beat. Although it is preferred to exclude beats which end in pace events, these intervals are not completely devoid of information concerning the supraventricular rhythm, and consequently it may be desirable to permit the use of such cycles in some instances. The other events and intervals 200-204 and 220 correspond to those of Fig.1.

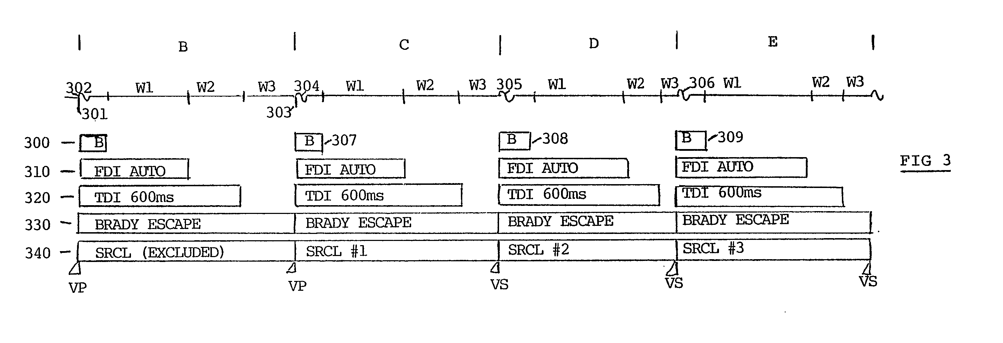

An example of an excluded cycle is shown in Fig. 3 as cycle B which begins with a ventricular pace event 301 event and ends with a ventricular pace event 303.

Preferably cardiac cycles which end in ventricular sense events are used to calculate the representative supraventricular rhythm cycle length (SRCL). Cycles which are included in the computation are shown in Fig. 3 as cycles C, D and E. For example, note that the ventricular depolarization 305 has fallen in window W3 of cycle C and therefore is likely to represent a beat of sinus origin. Likewise the depolarization shown at 306 also falls in window W3 of cycle D and is therefore presumed to be of supraventricular origin. Each of these beats may be used to compile a number which reflects a historic or average supraventricular rhythm cycle length SRCL AVE which is representative of the recently preceding heart rate.

The number of sample cycles used to compute SRCL AVE is somewhat arbitrary but should be large enough to represent the rhythm prior to the onset of any arrhythmia. However, the number of cycles must be small enough that the value of the SRCL AVE reflects the rhythm which just precedes the onset of the arrhythmia. At the present time, a minimum of eight sample cycles are regarded as sufficient to achieve these objectives, but any number from 4 to 47, for example, may be used.

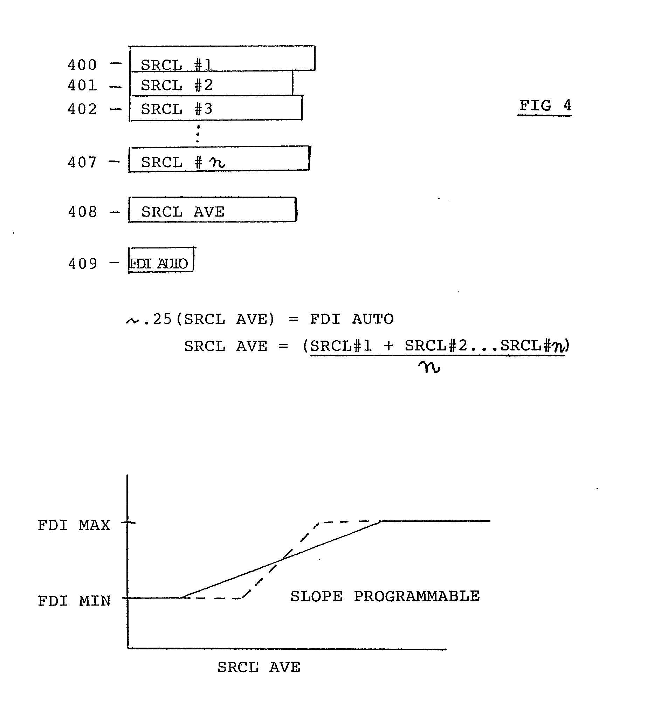

Various weighting or averaging schemes may be employed to derive the representative supraventricular rhythm cycle length from the eight sample cycles. At present, a running average is regarded as the most attractive. In this scheme, the acceptance of a new sample cycle results in the discarding of the oldest sample cycle so that the "average" is based on the most recent sample cycles. This computation is shown pictorially in Fig. 4 where representative samples 400-407 are added together and divided by 8 to yield an average depicted as 408. In operation, the next sample cycle added to the running average of SRCL AVE would result in the exclusion of SRCL #1 400 from the value of SRCL AVE 408.

Once a suitable representative cycle length is computed, it is used to determine the fibrillation detection interval. Research results based on both human and canine data are set forth in the following Table.

| | CANINE | | HUMAN | |
|---|---|---|---|---|
| | mVFCL | mSRCL | mVFCL | mSRCL |
| mean | 151 ms | 600 ms | 208 ms | 836 ms |
| s.d. | 37 ms | 146 ms | 43 ms | 243 ms |
| max | 204 ms | 842 ms | 341 ms | 1583 ms |
| min | 102 ms | 404 ms | 148 ms | 593 ms |
| correlation coefficient | r = .80 | (p<.01) | r = .85 | (p<.001) |

In the data, the mean ventricular fibrillation cycle length of induced fibrillation is compared to the preceding mean sinus rhythm cycle length. The data set shows a correlation between these cycle lengths. It appears that the mVFCL is approximately one-fourth of the preceding mSRCL.

In the present invention, the physician may select an upper and lower bound for the fibrillation detection interval, as well as a slope of the relation between SRCL AVE and FDI AUTO. In operation, the size of the FDI AUTO will vary linearly on a beat-to-beat basis as a function of the SRCL AVE between defined limits as shown on Fig. 4.

The optimum function for computing FDI AUTO from SRCL AVE is the object of study. At present, a simple linear relationship appears to be adequate to exploit the correlation between the cycle lengths of sinus rhythm and fibrillation. As set forth in Fig. 4, the FDI should be set to approximately 25 to 30 percent of the representative SRCL.

## Claims

1. An implantable medical device for treating a patient's heart, of the type having at least one sense amplifier for detecting depolarizations of cardiac events for generating a sense event signal, and able to initiate at least one therapy regime for treating cardiac tissue in the event of detection of tachyarrhythmia; and including an arrhythmia detector comprising:
timing means responsive to said sense amplifier for determining the cycle length of cardiac cycles for generating a supraventricular rhythm sample cycle length value;
averaging means for computing a representative supraventricular rhythm cycle length from a collection of said supraventricular rhythm sample cycle length values;
means for computing a fibrillation detection interval length as a function of said representative supraventricular rhythm cycle length;
counter means for monitoring the number of sense event signals occurring in fibrillation detection intervals each beginning with a cardiac depolarisation and extending for said computed interval length; and
means responsive to said counter means for initiating said therapy regime.

2. The device of claim 1 wherein maximum and minimum values of said fibrillation detection interval length are established in said device, said maximum and minimum values being physician-programmable.

3. The device of claim 1 and 2 including means for blanking said sense amplifier during a blanking interval so that it does not generate a sense event signal, whereby said counter means counts depolarisations falling within a timing window extending from the end of the blanking interval to the end of the fibrillation detection interval.

4. The device of claim 3 including means for establishing a second timing window extending from the

4

end of the fibrillation detection interval to the end of a tachycardia detection interval of predetermined length and means for establishing a third timing window extending from the end of the tachycardia detection interval to the end of a bradycardia escape interval of predetermined length, and wherein said timing means is responsive only to sense events falling within said third timing window.

5. The device of any preceding claim wherein said sense amplifier is adapted to be coupled to the ventricular chamber of the patient's heart.

6. The device of any preceding claim wherein said collection of said supraventricular rhythm sample cycle length values includes four or more cycles but less than forty-eight cycles.

7. The device of claim 6 wherein said collection of said supraventricular rhythm sample cycle length values includes eight cycles.

8. The device of any preceding claim wherein said averaging means computes a running average of said sample cycle length values.

EP 0 358 303 A1

101  W1  W2  W3
102  103  104

100 — BLANK
110 — FDI 240-400ms
120 — TDI 240-600ms
130 — BRADYCARDIA ESCAPE INTERVAL 800ms

FIG 1 (PRIOR ART)

A

201  W1  W2  W3
202  203  204

MIN  MAX
200 — BLANK  211  212
210 — FDI AUTO
220 — TDI 240-600ms
230 — BRADYCARDIA ESCAPE INTERVAL 800ms
240 — SINUS RHYTHM CYCLE LENGTH COUNTER

FIG 2

EP 0 358 303 A1

|   | B |   |   | C |   |   | D |   |   | E |   |   |

302  W1  W2  W3 304  W1  W2  W3 305  W1  W2  W3 306 W1  W2  W3
301                  303

300 — [B]

310 — FDI AUTO         FDI AUTO —307      FDI AUTO —308      FDI AUTO —309

320 — TDI 600ms        TDI 600ms          TDI 600ms          TDI 600ms

330 — BRADY ESCAPE     BRADY ESCAPE       BRADY ESCAPE       BRADY ESCAPE

340 — SRCL (EXCLUDED)  SRCL #1            SRCL #2            SRCL #3

VP                     VP                 VS                VS                 VS

FIG 3

EP 0 358 303 A1

400 — SRCL #1
401 — SRCL #2
402 — SRCL #3
⋮
407 — SRCL # $n$

408 — SRCL AVE

409 — FDI AUTO

FIG 4

$\sim .25$ (SRCL AVE) = FDI AUTO

$$\text{SRCL AVE} = \frac{(\text{SRCL#1} + \text{SRCL#2}...\text{SRCL#}n)}{n}$$

FDI MAX

FDI MIN

SLOPE PROGRAMMABLE

SRCL AVE

## DOCUMENTS CONSIDERED TO BE RELEVANT | EP 89305786.9

| Category | Citation of document with indication, where appropriate, of relevant passages | Relevant to claim | CLASSIFICATION OF THE APPLICATION (Int. Cl.4) |
|---|---|---|---|
| A | AU - B - 68 641/81 (TELECTRONICS)  * Page 3, line 28 - page 4, line 30; claims 1,2,4,6; fig. 10-14 * | 1-3,6 | A 61 N 1/365 A 61 B 5/04 |
| A | EP - A1 - 0 218 006 (SIEMENS)  * Abstract; claim 2 * | 1,3,4 | |
| A | EP - A1 - 0 198 087 (KABUSHIKI KAISHA)  * Abstract; fig. 1-3,5 * | 1 | |
| A | EP - A1 - 0 150 246 (INSTYTUT PSYCHONEUROLOGICZNY)  * Abstract; claim 1 * | 1,3,4 | |
| A | US - A - 4 712 556 (BAKER)  * Abstract; fig. 2-9 * | 1 | |
| A | US - A - 4 390 021 (SPURREL)  * Abstract; fig. 11-14 * | 1 | TECHNICAL FIELDS SEARCHED (Int. Cl.4)  A 61 N A 61 B |
| D,A | US - A - 4 384 585 (ZIPES)  * Abstract; column 1, line 55 - column 2, line 3 * | 1,5 | |
| A | US - A - 4 354 497 (KAHN)  * Abstract; column 1, line 57 - column 2, line 10 * | 1 | |

The present search report has been drawn up for all claims

| Place of search | Date of completion of the search | Examiner |
|---|---|---|
| VIENNA | 22-11-1989 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82